# EUROPEAN PATENT APPLICATION

(11) **EP 2 033 945 A1**
(43) Date of publication of application: **11.03.2009**
(21) Application number: 07115858.8
(22) Date of filing: 06.09.2007
(51) Int. Cl.: C07C 209/20, C07C 211/63, C10L 1/22

(54) **Quaternary ammonium salts**

(71) Applicant: Infineum International Limited, Abingdon Oxfordshire OX13 6BB (GB)
(72) Inventor: Tack, Robert Dryden, Abingdon, Oxfordshire OX13 6BB (GB)
(74) Representative: Capaldi, Michael Joseph

(57) **Abstract**

Halogen- and sulphur-free quaternary ammonium salts of carboxylic acids, useful as cold flow additives in middle distillate fuels, are made by reacting, in the presence of an acid catalyst: (A) a halogen- and sulphur-free tertiary amine that carries at least one alkyl group containing 8 to 40 carbon atoms; and (B) a halogen- and sulphur-free C₁ to C₄ alkyl ester of a carboxylic acid that has a pKₐ of 3.5 or less.

## Description

### FIELD OF THE INVENTION

This invention relates to a process for the preparation of halogen- and sulphur-free ammonium salts of carboxylic acids.

### BACKGROUND OF THE INVENTION

The art describes processes for making quaternary ammonium compounds. One such process involves alkylating a tertiary amine to the quaternary stage using as alkylating agent an ester of a strong mineral acid or an alkyl halide. Another such process involves reacting the salt of a tertiary amine and an acid with an epoxide compound.

The former known process has the disadvantage of introducing a counter-ion (anion) such as chloride, bromide, sulphate or methylsulphate, which may cause the quaternary ammonium compound to be corrosive. Additionally or alternatively, the presence of such anions may not be permitted or may be undesirable for environmental reasons. For example, certain legislations severely limit the levels of chlorine and sulphur in hydrocarbon fuels.

The latter known process involves two stages, tertiary amine salt preparation followed by epoxide compound treatment, and necessarily introduces a hydroxyalkyl residue into the quaternary ammonium compound. Generally, in such a process, for example as described in US-A-4,421,932 ('932), US-A-4,678,605 ('605) and US-A-4,814,108, no more than two of the four hydrocarbyl groups on the product quaternary salt have long chains, one long-chain group being from the epoxide compound and one long-chain group only being from the tertiary amine. Attempts to carry out the process of '932, but substituting acetic acid for hydrochloric acid (use of carboxylic acids is described in '605), have shown the following: triethylamine reacts effectively with epoxybutane; dimethyl-dodecylamine is poorly converted when reacted with epoxytetradecane; and methyl-di-hydrogenated tallow amine does not significantly react with epoxytetradecane.

### SUMMARY OF THE INVENTION

The present invention meets the above problems by providing a one-stage process in which a tertiary amine is reacted with an ester of a defined carboxylic acid.

Thus, in one aspect, the invention provides a process for the preparation of a halogen- and sulphur-free quaternary ammonium salt of a carboxylic, preferably polycarboxylic, acid which comprises reacting, in the presence of an acid catalyst: (A) a halogen- and sulphur-free tertiary amine that carries at least one alkyl group containing from 8 to 40 carbon atoms; and (B) a halogen- and sulphur-free C₁ to C₄ alkyl ester of a carboxylic, preferably polycarboxylic, acid that has a pKₐ of 3.5 or less, preferably 3 or less, more preferably 2.5 or less.

The anion is non-corrosive and hydroxyl groups are not present in the long chains. Also, as evidenced in the data herein, quaternisation may be successfully carried out on tertiary amines bearing more than one long-chain alkyl group.

In a second aspect, the invention provides a halogen- and sulphur-free quaternary ammonium salt of the formula [NR₂R¹³R¹⁴]X wherein: R represents a methyl, ethyl or propyl group; R¹³ represents an alkyl group containing 8 to 40 carbon atoms; R¹⁴ represents an alkyl group containing up to 40 carbon atoms; and X represents a monovalent carboxylate anion.

In a third aspect, the invention provides a middle-distillate fuel oil composition comprising a minor amount of a halogen- and sulphur-free quaternary ammonium salt of a carboxylic acid of the second aspect of the invention or obtained by or obtainable by the process of the first aspect of the invention.

### DETAILED DESCRIPTION OF THE INVENTION

Features of the invention, applicable where appropriate to various aspects of the invention, will now be described in more detail.

### PROCESS

The process is, as stated above, acid-catalysed, the acid preferably being a carboxylic acid such as neodecanoic acid, tallow acid or rapeseed acid. Importantly, the acid used for catalysis is sulphur- and halogen-free so that atoms of those elements are not introduced into the quaternary salt product by way of the catalyst.

Conveniently, the process is carried out at elevated temperature such as in the range of 100 to 150°C and for one or more hours. However, the combination of temperature and time may be varied and chosen by means of routine experimentation.

The ester is most preferably used in molar excess, suitably a large excess, ester remaining after completion of the process being removable by evaporation or distillation.

The tertiary amine reactant (A) preferably carries a segment of the formula NR¹³R¹⁴ where R¹³ represents an alkyl group containing from 8 to 40 carbon atoms and R¹⁴ represents an alkyl group containing up to 40, more preferably from 8 to 40, carbon atoms. Surprisingly, quaternary ammonium salts may be made from tertiary amines that carry two long chain alkyl groups, notwithstanding possible steric hindrance problems. R¹³ and R¹⁴ may be straight chain or branched, and/or may be the same or different; advantageously, each of R¹³ and R¹⁴ represents a C₁₂ to C₂₄ straight-chain alkyl group. However, quaternary ammonium salts have been successfully made, employing the present invention, from tertiary amines that carry only one long-chain alkyl group, e.g. where R¹⁴ represents a methyl, ethyl or propyl group.

Suitably, the segment NR¹³R¹⁴ is derived from a secondary amine such as dioctadecylamine, di-cocoamine, di-hydrogenated tallow amine and methylbehenylamine. Amine mixtures are also suitable such as those derived from natural materials, preferably a secondary hydrogenated tallow amine, the alkyl groups of which are derived from hydrogenated tallow fat composed of approximately 4% C₁₄, 31% C₁₆ and 59% C₁₈ alkyl groups, where the percentages are by weight.

As an example of tertiary amine that may be used, there may be mentioned a tertiary amine of the formula NR¹³R¹⁴R where R¹³ and R¹⁴ are defined as above and R represents a methyl, ethyl or propyl group, methyl being preferred.

As examples of the carboxylic acid of the ester reactant (B) there may be mentioned oxalic acid (1.2), phthalic acid (2.9), salicylic acid (3.0), maleic acid (1.8), malonic acid (2.8), citric acid (3.1) and 2,4,6-trihydroxybenzoic acid (1.7). pKₐ values are indicated in brackets and are obtained from "Dissociation Constants of Organic Acids in Aqueous Solutions", page D161, CRC Handbook of Chemistry and Physics, 68th edition, 1987 - 1988. Dicarboxylic acids are preferred, most preferably oxalic acid. The ester is preferably a methyl ester and is more preferably dimethyl oxalate.

Where the carboxylic acid is polybasic in this invention, the dissociation constant referred to is the first dissociation constant.

### SALTS

The salts of the invention are effective as wax anti-settling additives (WASA) in fuel oils whereby they cause wax crystals crystallising in the oil to be retained in suspension thereby reducing settling and thus assisting in prevention of blockages. Some of the salts, such as those of the above general formula where R and R¹⁴ each represent methyl groups and R¹³ represents, for example, a dodecyl group, may be useful as cationic detergents. Others, such as those where R represents a methyl group and R¹³ and R¹⁴ each represent alkyl groups derived from hydrogenated tallow fat, may be useful as fabric conditioners.

### THE FUEL OIL

The fuel oil is a petroleum-based fuel oil in the form of a middle distillate fuel oil, generally boiling within the range of from 110 to 500, e.g. 150 to 400,°C.

The invention is applicable to middle distillate fuel oils of all types, including the broad-boiling distillates, i.e., those having a 90%-20% boiling temperature difference, as measured in accordance with ASTM D-86, of 100°C or more.

The fuel oil may comprise atmospheric distillate or vacuum distillate, cracked gas oil, or a blend in any proportion of straight-run and thermally-and/or catalytically-cracked distillates. The most common petroleum distillate fuels are kerosene, jet fuels, diesel fuels, heating oils and heavy fuel oils. The heating oil may be a straight atmospheric distillate, or may also contain vacuum gas oil or cracked gas oil or both. The fuels may also contain major or minor amounts of components derived from the Fischer-Tropsch process. Fischer-Tropsch fuels, also known as FT fuels, include those that are described as gas-to-liquid fuels, coal and/or biomass conversion fuels. To make such fuels, syngas (CO + H₂) is first generated and then converted to normal paraffins and olefins by the Fischer-Tropsch process. The normal paraffins may then be modified by processes such as catalytic cracking/reforming or isomerisation, hydrocracking and hydroisomerisation to yield a variety of hydrocarbons such as iso-paraffins, cyclo-paraffins and aromatic compounds. The resulting FT fuel can be used as such or in combination with other fuel components and fuel types such as those mentioned in this specification. The above-mentioned low temperature flow problem is most usually encountered with diesel fuels and with heating oils. The invention is also applicable to fuel oils containing fatty acid methyl esters derived from vegetable oils, for example, rapeseed methyl ester, either used alone or in admixture with a petroleum distillate oil.

The fuel oil is preferably a low sulphur-content fuel oil. Typically, the sulphur content of the fuel oil is less than 500ppm (parts per million) by weight. Preferably, the sulphur content of the fuel is less than 100, for example less than 50, ppm. Fuel oils with even lower sulphur contents, for example less than 20ppm or less than 10ppm, are also suitable.

### TREAT RATES

Typically, the amount of quaternary salt effective as a wax anti-settling additive is in the range of 10 - 500, preferably 10 - 200, more preferably 10 - 100, ppm by weight based on the weight of the fuel oil.

### OTHER ADDITIVES

It is commonplace in the art to use polar nitrogen compounds effective as a wax anti-settling additives in combination with other additional cold-flow improving additives. Suitable materials will be well known to those skilled in the art and include, for example, ethylene-unsaturated ester copolymers such as ethylene:vinyl acetate copolymers and similar polymers. The present invention contemplates the addition of such additional cold-flow improving additives, their application in terms of treat rate also being well known to those skilled in the art.

### EXAMPLES

The invention will now be described in the examples, which are not intended to limit the scope of the claims hereof.

### PREPARATION OF QUATERNARY AMMONIUM SALTS

### Example 1

Methyl-di-hydrogenated tallow amine (100g; 0.2 mole) and dimethyl oxalate (100g; 0.85 mole) were mixed together with neodecanoic acid (2g; 1 mass %) as catalyst. The mixture was heated for four hours at 120°C to effect quaternisation. The product was stripped under vacuum on a rotary evaporator at 100 to 120°C to remove excess dimethyl oxalate. Hnmr analysis of the resulting salt indicated that all of the starting tertiary amine had been converted to di-methyl-di-hydrogenated tallow ammonium monomethyl oxalate.

### Examples 2-6 and A and B

Further quaternary ammonium salts were made by a process in which a dimethyldodecylamine (10g) and an ester (20g) were mixed with neodecanoic acid (0.5g) as catalyst. The mixture, which contained 3 to 4 moles of ester per mole of tertiary amine, was heated for 4 hours at 120°C to effect quarternisation and then stripped at 100°C on a hot water bath. The percentage of non-volatile material present was determined by weighing the product before and after treatment at 100°C under vacuum (0.1 to 0.5 mmHg), and the yield (conversion) determined by mass balance.

The esters used in Examples 2-6 were those of carboxylic acids having a pKₐ of 3.5 or less, i.e. within the scope of this invention, and, by way of comparison in Examples A and B, methyl palmitate and methyl benzoate. Palmitic acid is not water-soluble, but its closest analogue, butyric acid whose pKₐ is measurable, has a pKₐ of 4.8. The theoretical pKₐ of palmitic acid would be expected to be similar, i.e. outside the scope of this invention. In comparison Example A, the weight ratio of ester to tertiary amine was 50/10 g to ensure a comparable molar ratio of about 4/1. The ester used in comparison Example B is of benzoic acid, which has a pKₐ of 4.2, i.e. outside the scope of the invention. In comparison Example B, the weight ratio of ester to tertiary amine was 30/10g to ensure a comparable molar ratio.

The esters and the results are tabulated below, where the Examples of the invention are identified by numbers and the comparison examples by letters.

| Examples | Ester | Conversion (%) |
|---|---|---|
| 2 | dimethyl oxalate | 97 |
| 3 | diethyl oxalate | 50 |
| 4 | dimethyl maleate | 60 |
| 5 | dimethyl phthalate | 67 |
| 6 | dimethyl fumarate | 32 |
| A | methyl palmitate | 0 |
| B | methyl benzoate | 0 |

It is seen that each of the examples of the invention gave rise to conversation to the corresponding quaternary salt. In contrast, no conversation to the quaternary salt occurred in the comparison examples.

### TESTS

Quaternary ammonium compounds of the invention were tested in middle distillate fuels in combination with cold flow improver additives known in the art. The compounds were found to improve Cold Filter Plugging Print (CFPP) performance in comparison with otherwise identical fuel compositions lacking the compounds.

## Claims

1. A process for the preparation of a halogen- and sulphur-free quaternary ammonium salt of a carboxylic acid which comprises reacting, in the presence of an acid catalyst:
(A) a halogen- and sulphur-free tertiary amine that carries at least one alkyl group containing from 8 to 40 carbon atoms; and
(B) a halogen- and sulphur-free C₁ to C₄ alkyl ester of a carboxylic acid that has a pKₐ of 3.5 or less.

2. A process as claimed in claim 1 wherein the tertiary amine carries a segment of the formula NR¹³R¹⁴ where R¹³ represents an alkyl group containing from 8 to 40 carbon atoms, and R¹⁴ represents an alkyl group containing up to 40 carbon atoms.

3. A process as claimed in claim 2 wherein the tertiary amine has the formula NR¹³R¹⁴R where R represents a methyl, ethyl or propyl group.

4. A process as claimed in claim 2 or 3 wherein each of R¹³ and R¹⁴ represents a C₁₂ to C₂₄ straight-chain alkyl group.

5. A process as claimed in claim 4 wherein R¹³ and optionally R¹⁴ represent alkyl groups derived from hydrogenated tallow fat.

6. A process as claimed in claim 3 wherein R¹³ represents a methyl, ethyl or propyl group and R¹⁴ represents a C₁₂ to C₂₄ straight-chain alkyl group.

7. A process as claimed in any of claims 1 to 6 wherein the carboxylic acid is a dicarboxylic acid such as oxalic acid.

8. A process as claimed in any of claims 1 to 7 wherein the ester is a methyl ester.

9. A process as claimed in any of claims 1 to 8 wherein the acid catalyst is a monocarboxylic acid that is halogen-free and sulphur-free.

10. A process for the preparation of a halogen- and sulphur-free quaternary ammonium salt of the monomethyl ester of oxalic acid which comprises reacting, in the presence of an acid catalyst:
(A) a halogen- and sulphur-free tertiary amine that carries three alkyl groups, one or two being C₁₂ to C₂₄ straight-chain alkyl groups and the balance being methyl; and
(B) di-methyl oxalate.

11. A halogen- and sulphur-free quaternary ammonium salt of the formula [NR₂R¹³R¹⁴] X wherein:
R represents a methyl, ethyl or propyl group;
R¹³ represents an alkyl group containing 8 to 40 carbon atoms;
R¹⁴ represents an alkyl group containing up to 40 carbon atoms; and
X represents a monovalent carboxylate anion.

12. A middle-distillate fuel oil composition comprising a minor amount of a halogen- and sulphur-free quaternary ammonium salt of a carboxylic acid of claim 11 or obtained by or obtainable by a process as claimed in any one of claims 1 to 10.
